# EUROPÄISCHE PATENTSCHRIFT

(11) **EP 3 716 920 B1**
(45) Veröffentlichungstag und Bekanntmachung des Hinweises auf die Patenterteilung: **12.07.2023**
(21) Anmeldenummer: 18812144.6
(22) Anmeldetag: 30.11.2018
(51) Int. Cl.: A61F 5/01

(54) **ORTHESE, SYSTEM UND VERFAHREN ZUM ANPASSEN EINER ORTHESE**
ORTHOSIS, SYSTEM AND PROCEDURE FOR ADAPTING AN ORTHOSIS
ORTHÈSE, SYSTÈME ET PROCÉDURE POUR ADAPTER UNE ORTHÈSE

(30) Priorität: 01.12.2017 DE 102017128618
(43) Veröffentlichungstag der Anmeldung: 07.10.2020
(73) Patentinhaber: Ottobock SE & Co. KGaA, 37115 Duderstadt (DE)
(72) Erfinder: SIEWERT, Gordon, 37083 Göttingen (DE)
(74) Vertreter: Gramm, Lins & Partner Patent- und Rechtsanwälte PartGmbB
(86) Internationale Anmeldenummer: PCT/EP2018/083134
(87) Internationale Veröffentlichungsnummer: WO 2019/106144

(56) Entgegenhaltungen:
- EP-A1- 1 410 774
- US-A- 4 433 679
- US-A- 5 954 621
- US-A1- 2014 308 065

## Beschreibung

Die Erfindung betrifft eine Orthese mit einem Oberteil und einem Unterteil, die über eine erste Gelenkeinrichtung verschwenkbar um eine Schwenkachse miteinander gekoppelt sind, wobei an dem Oberteil und/oder dem Unterteil Einrichtungen zur Aufnahme eines Körperteils oder einer Gliedmaße oder Festlegung der Orthese an einem Körperteil oder einer Gliedmaße angeordnet sind, ein System aus einer Orthese und einem Funktionselement sowie ein Verfahren zum Anpassen einer Orthese an einen Patienten.

Orthesen werden an eine Gliedmaße angelegt und weisen in der Regel Schienen oder Schalen mit Einrichtungen zum Festlegen der jeweiligen Schienen oder Schalen an der Gliedmaße auf. Die Schienen oder Schalen sind über eine Gelenkeinrichtung miteinander verbunden, so dass eine gelenkübergreifende Anordnung der Orthese an der Gliedmaße stattfinden kann. Über eine Orthese können Bewegungen geführt, Verschwenkwinkel um eine Gelenkachse begrenzt, Verschwenkbewegungen verhindert oder eine Ausrichtung von Gliedmaßen zueinander unterstützt oder festgelegt werden. Darüber hinaus können Orthesen mit Dämpferelementen versehen sein, um Verschwenkbewegungen um die Gelenkachse zu dämpfen. Die Dämpfereinrichtungen können mit einer Steuerung versehen sein, so dass in Abhängigkeit von Belastungen, Schwenkwinkeln oder Sensordaten eine veränderte Dämpfung in Flexionsrichtung oder Extensionsrichtung bereitgestellt werden kann.

Es besteht die Möglichkeit, dem Oberteil und dem Unterteil einen Kraftspeicher zuzuordnen, so dass über eine Vorspannung einer Feder oder eines Elastomerelementes oder eines hydraulischen oder pneumatischen Kraftspeichers beispielsweise ein Widerstand gegen eine Verschwenkbewegung in einer ersten Richtung bereitgestellt und erhöht wird, wobei durch eine Entspannung des Kraftspeichers die entgegengesetzte Bewegung unterstützt wird. In den Gelenkeinrichtungen können Endanschläge zur Einstellung der jeweiligen Verschwenkwinkel verstellbar ausgebildet sein, ein Dämpfer zur Vermeidung eines ungedämpften Anschlages in einen Endanschlag kann ebenfalls vorgesehen sein.

Darüber hinaus existieren Orthesen mit aktiven Antrieben, bei denen über beispielsweise über einen Elektromotor eine Verlagerung des Oberteils relativ zu dem Unterteil um die Schwenkachse aktiv bewirkt wird. Die Orthesen können nur ein Gelenk übergreifen, beispielsweise das Kniegelenk oder das Knöchelgelenk, ebenfalls ist es möglich und vorgesehen, dass die Orthese mehrere Gelenke übergreift, beispielsweise das Kniegelenk und das Knöchelgelenk oder auch das Hüftgelenk, das Kniegelenk und das Knöchelgelenk. Bei einer Orthese, die mehrere natürliche Gelenke übergreift, ist die jeweils proximale Komponente das Oberteil und die distale Komponente das Unterteil. Bei einer Knie-Knöchel-Orthese ist somit eine Unterschenkelschiene das Unterteil, hinsichtlich des knieübergreifenden Anteils, da die Unterschenkelschiene distal zu einer Oberschenkelschiene angeordnet ist. Hinsichtlich einer Fußschiene oder Fußschale ist die Unterschenkelschiene dann ein Oberteil, während die Fußschiene oder Fußschale, die über ein Orthesen-Knöchelgelenk mit der Unterschenkelschiene verbunden ist, das Unterteil ausbildet.

Bei hochbelasteten Orthesen, beispielsweise bei Knie-Knöchel-Fuß-Orthesen, die als KAFO (knee-ankle-foot orthosis) abgekürzt werden, werden im Kniebereich bilateral Gelenke vorgesehen, um eine Orthese auszugestalten. Dabei ist sowohl auf der medial als auch auf der lateralen Seite des Kniegelenkes eine Gelenkeinrichtung angeordnet, die jeweils eine Oberschenkelschiene und eine Unterschenkelschiene aufweisen, die miteinander verbunden sind. Problematisch bei einer bilateralen Anordnung von Schienen an einem Bein ist die schwierige Anpassbarkeit an verschiedene Beinumfänge, die störende mediale Gelenkeinrichtung, die mit dem unversorgten Bein kollidieren kann, sowie eine aufwendige Anpassbarkeit, insbesondere bei Testversorgungen.

Solche bilateralen Orthesen sind beispielsweise aus der US 5,954,621 A und der EP 1 410 774 A1 bekannt. Beide beschreiben Knieorthesen mit einem medialen und einem lateralen Orthesengelenk. In der US 5,924,621 A, die den Oberbegriff des Anspruchs 1 offenbart, kann den Orthesengelenken jeweils ein Steuermodul zugeordnet werden, welche beispielsweise Reibungselemente zum Bereitstellen eines Schwenkwiderstands aufweisen. In der EP 1 410 774 A1 kann einem der Orthesengelenke eine Motoreinheit zum aktiven Verschwenken der Orthesenteile relativ zueinander zugeordnet sein.

Aufgabe der vorliegenden Erfindung ist es daher, eine Orthese, eine System aus einer Orthese und einem Funktionselement und ein Verfahren bereitzustellen, die die Nachteile aus dem Stand der Technik nicht aufweisen. Erfindungsgemäß wird diese Aufgabe durch eine Orthese mit den Merkmalen des Hauptanspruches sowie ein System und ein Verfahren mit den Merkmalen der nebengeordneten Ansprüche gelöst. Vorteilhafte Ausgestaltungen und Weiterbildungen der Erfindung sind in den Unteransprüchen, der Beschreibung sowie den Figuren offenbart.

Die erfindungsgemäße Orthese mit einem Oberteil und einem Unterteil, die über eine erste Gelenkeinrichtung schwenkbar um eine Schwenkachse miteinander gekoppelt sind, wobei an dem Oberteil und/oder Unterteil Einrichtungen zur Aufnahme eines Körperteils oder einer Gliedmaße oder bei Festlegung der Orthese an einem Körperteil oder einer Gliedmaße angeordnet sind, sieht vor, dass das Oberteil und das Unterteil jeweils nur einseitig medial oder lateral neben der zu versorgenden Gliedmaße angeordnet ist und an dem Oberteil einseitig, insbesondere lateral ein oberes Stützelement und an dem Unterteil einseitig, insbesondere lateral ein unteres Stützelement festgelegt sind, die über eine zweite Gelenkeinrichtung schwenkbar um die Schwenkachse miteinander verbunden sind, wobei an dem Oberteil und/oder dem Unterteil eine Lagerplatte mit zumindest einer Befestigungseinrichtung zur Festlegung der zweiten Gelenkeinrichtung befestigt oder ausgebildet ist. Durch die Anordnung der beiden Stützelemente einseitig, insbesondere lateral an dem Oberteil und dem Unterteil, gegebenenfalls auch an Schienenteilen oder Komponenten, die an dem Oberteil und Unterteil angeordnet oder ausgebildet sind, wird eine verbesserte Aufnahme der in der Orthese entstehenden Torsionsmomente sowie Momente in einer Frontalebene ermöglicht. Die Funktionsfähigkeit der Orthese ist dabei nicht eingeschränkt, da die zweite Gelenkeinrichtung, die das obere und untere Stützelement schwenkbar miteinander verbindet, eine Verschwenkung um eine gemeinsame Schwenkachse der beiden Gelenkeinrichtungen ermöglicht. Die Schwenkachsen der Gelenkeinrichtungen sind koaxial, wobei die Schwenkachse bevorzugt koaxial zu der natürlichen Gelenkachse oder einer Kompromissgelenkachse verläuft, um eine möglichst unbeeinträchtigte Bewegung der natürlichen Gliedmaße zu ermöglichen. Insbesondere die zweite Gelenkeinrichtung kann eine virtuelle Gelenkachse aufweisen, es ist daher nur notwendig, dass das obere und untere Stützelement um eine gemeinsame Schwenkachse mit dem Oberteil und Unterteil verschwenken können. An dem Oberteil und/oder dem Unterteil ist eine Lagerplatte befestigt oder ausgebildet, die zumindest eine Befestigungseinrichtung aufweist, an der die zweite Gelenkeinrichtung festgelegt werden kann. Über die Lagerplatte und die Befestigungseinrichtung ist es möglich, unterschiedliche Gelenkeinrichtungen an dem Oberteil und/oder dem Unterteil zu befestigen, ohne Anpassungen an der zweiten Gelenkeinrichtung, insbesondere an dem oberen Stützelement und dem unteren Stützelement, vornehmen zu müssen. Dadurch kann der modulare Aufbau der Orthese erleichtert werden und insbesondere aus einer Vielzahl unterschiedlicher zweiter Gelenkeinrichtungen eine Auswahl getroffen werden, um beispielsweise zu Testzwecken eine Montage vorzunehmen. Insbesondere wenn mehrere Befestigungseinrichtungen an unterschiedlichen Stellen an der Lagerplatte angeordnet oder ausgebildet sind, wird die Montage unterschiedlicher zweiter Gelenkeinrichtungen erleichtert. Es ist nicht mehr notwendig, unmittelbar an dem Oberteil oder dem Unterteil das obere und untere Stützelement festzulegen, vielmehr kann eine Festlegung im Wesentlichen unabhängig von der Ausgestaltung des Oberteils und/oder des Unterteils erfolgen. Die Befestigungseinrichtungen können verlagerbar an der Lagerplatte angeordnet sein. Über verlagerbare Befestigungseinrichtungen, beispielsweise Schraubeinsätze, Bolzen, feststellbare Gleitführungen oder ähnliches, kann die Anpassbarkeit an unterschiedliche zweite Gelenkeinrichtungen vergrößert werden. Die Lagerplatte als solche muss nicht eben ausgebildet sein, sie kann ebenfalls eine Krümmung aufweisen. Lagerplatten können sowohl an dem Oberteil als auch an dem Unterteil angeordnet sein, alternativ ist eine Lagerplatte entweder an dem Oberteil oder an dem Unterteil angeordnet und dient zur Festlegung der zweiten Gelenkeinrichtung. Bei nur einer Lagerplatte wird das jeweils andere Stützelement, das nicht an der Lagerplatte festgelegt ist, an dem Oberteil beziehungsweise dem Unterteil festgelegt. Die Lagerplatte ist ein Adapter, der an der ersten Gelenkeirichtung angeordnet ist und über den die Befestigung der zweiten Gelenkeinrichtung erfolgen kann oder erleichtert wird.

Aufgrund der Anordnung der zusätzlichen Stützelemente einseitig, insbesondere lateral zu dem Oberteil und dem Unterteil und der sich damit ergebenden einseitigen, insbesondere lateralen Anordnung des Oberteils und des Unterteils neben der natürlichen Gliedmaße ist der gegenüberliegende, insbesondere mediale Bereich der Gliedmaße, insbesondere bei der Anwendung einer Orthese als Orthese einer unteren Extremität, frei, so dass ein schmaler Aufbau erreicht und bei einer lateralen Anordnung zudem keinerlei Kollisionen von Orthesenkomponenten mit der kontralateralen Seite oder dem Torso auftreten können. Sofern beide Gliedma-ßen mit einer solchen Orthese mit lateraler Anordnung ausgerüstet werden, insbesondere bei einer Versorgung der unteren Gliedmaßen, ist mehr Platz zum Durchschwingen der unteren Gliedmaße auf der medialen Seite vorhanden, was eine Erhöhung des Tragekomforts mit sich bringt.

Die Orthese kann über Einrichtungen zur Aufnahme eines Körperteils an einer Gliedmaße oder Befestigungseinrichtungen zur Festlegung der Orthese an einem Körperteil oder einer Gliedmaße verfügen. Häufig werden Orthesen über ein Gurtsystem oder eine Kombination aus einer Schalenaufnahme mit einem Gurtsystem an dem Orthesennutzer festgelegt. Eine einseitige, insbesondere nur laterale Anordnung der doppelten Gelenkeinrichtung und damit der Schienen oder der Schalen, die sich von der Gelenkeinrichtung weg entlang der Gliedmaße erstrecken, erleichtert die Anpassung der Orthese an verschiedene Umfänge von Gliedmaßen, so dass eine solche Orthese sehr gut als eine sogenannte Testorthese eingesetzt werden kann, an der die endgültige Zusammenstellung der einzelnen Komponenten für eine optimale Patientenversorgung angeordnet oder ausgewechselt werden können.

Eine Weiterbildung der Erfindung sieht vor, dass die erste Gelenkeinrichtung und die zweite Gelenkeinrichtung lateral zueinander beabstandet sind. Durch den Abstand der Gelenkeinrichtungen zueinander ergibt sich eine umlaufende Rahmenkonstruktion, über die Torsionsmomente entlang der Längserstreckung der Orthese verbessert aufgenommen werden können. Ebenso können die Momente in der frontalen Ebene bei einer vergleichsweise breiten Ausgestaltung aufgrund eines Abstandes zwischen den beiden Gelenkeinrichtungen verbessert aufgenommen werden, so dass sich eine stabilere Ausgestaltung der Orthese ergibt, auch wenn die Gelenkeinrichtungen nur einseitig lateral an einer Gliedmaße angeordnet sind.

Die Stützelemente können lösbar aneinander festgelegt und über die zweite Gelenkeinrichtung miteinander verbunden sein, um die einzelnen Komponenten leichter austauschen zu können. So können unterschiedliche obere Stützelemente mit nur einem unteren Stützelement lösbar verbindbar sein, so dass verschiedene Geometrien, Abmessungen oder Anbindungen in dem oberen Stützelement bereitgestellt und einfach ausgewechselt werden können, wenn dies zur Anpassung an den jeweiligen Patienten notwendig ist. Dadurch ist es möglich, mit einer Basisorthese und unterschiedlichen oberen und unteren Stützelementen, die miteinander kombinierbar sind, eine Vielzahl von individuell anpassbaren Orthesen bereitzustellen.

An dem Oberteil und dem Unterteil können Befestigungseinrichtungen zur lösbaren Befestigung von Schienen oder Einrichtungen zur Aufnahme eines Körperteils oder einer Gliedmaße ausgebildet oder angeordnet sein. Die Befestigungseinrichtungen sind bevorzugt sogenannte Schienenkästen oder Aufnahmen für Schienen, die in dem Oberteil formschlüssig festgelegt sind, beispielsweise durch Schrauben oder durch ähnliche lösbare Formschlusselemente. Dadurch ist es möglich, unterschiedliche Schienen oder Einrichtungen zur Aufnahme eines Körperteils oder einer Gliedmaße, beispielsweise Schalen, Fußstützen oder dergleichen, an dem Oberteil oder Unterteil variabel zu befestigen, um eine einfache Anpassung an die Bedürfnisse der Nutzer zu erreichen.

An dem oberen Stützelement und dem unteren Stützelement können Aufnahmen für einen Dämpfer, einen Antrieb, eine Steuerungseinrichtung und/oder einen Sensor angeordnet oder ausgebildet sein, um über die Stützelemente eine Erweiterung der Funktionalität der Orthese bereitstellen zu können. Das obere und untere Stützelement dient dabei als Träger für weitere Komponenten, beispielsweise Dämpfereinrichtungen oder Antriebe, um die Verschwenkbewegung der Orthese zu beeinflussen. Es können rein passive Zusatzkomponenten wie Dämpfereinrichtungen oder Federn über die Stützelemente an die Orthese angebracht werden, so dass die Stützelemente als Modulträger eingesetzt werden. Die Dämpfereinrichtung kann einstellbar sein, insbesondere kann eine Einstellbarkeit aufgrund von Sensordaten erfolgen, wozu innerhalb der Dämpfereinrichtung eine Verstelleinrichtung mit einer Steuerungseinrichtung vorhanden sein muss, über die auf Grundlage von Sensordaten eine Verstellung der Dämpfung stattfindet. Ebenfalls kann ein Antrieb, insbesondere ein motorischer Antrieb oder ein durch eine Pumpe angetriebenen Aktuator an der Orthese über das obere und untere Stützelement befestigt werden, so dass über einen motorischen Antrieb eine Bewegung der Gliedmaße unterstützt oder überhaupt ermöglicht wird. Der motorische Antrieb kann gleichzeitig als Dämpfer eingesetzt werden. Die Stützelemente verfügen über geeignete Aufnahmen für die einzusetzenden Komponenten, die an den Stützelementen angeschraubt oder auf andere Art und Weise kraftüberragend und insbesondere formschlüssig daran gekoppelt werden können, insbesondere Federspeicher, Vorbringereinrichtungen, Freigabe- und Verriegelungseinrichtungen, Aktuatoren, Energiespeicher, Pumpen, Motoren, Dämpfer Steuerungseinrichtungen und/oder Sensoren.

Die Stützelemente sind bevorzugt lösbar über die Lagerplatte an dem Oberteil und dem Unterteil befestigt, um eine leichte Austauschbarkeit der Stützelemente zu erreichen. Aufgrund der lateralen Anordnung sowohl des oberen als auch des unteren Stützelementes kann ein Austausch auch bei angelegter Orthese leicht erfolgen, um eine Anpassung an den jeweiligen Nutzer zu erleichtern. Die Lagerplatte selbst kann bevorzugt auswechselbar an dem Oberteil oder Unterteil befestigt sein, um unterschiedliche Lagerplatten montieren zu können, die unterschiedliche Befestigungseinrichtungen aufweisen können oder Befestigungseinrichtungen an unterschiedlichen Stellen oder in voneinander abweichenden Anordnungen aufweisen.

Das obere und/oder untere Stützelement kann zu der Schwenkachse winkeleinstellbar ausgebildet sein, so dass eine Verkippung bei einer gestreckten Stellung der Orthese in der Frontalebene möglich ist. Diese Verkippung beeinträchtigt nicht die Koaxialität der Schwenkachsen und kann notwendig sein, um eine optimierte Anpassung an die jeweiligen Nutzungsbedingungen vornehmen zu können.

Die Orthese ist bevorzugt als unilaterale Orthese einer unteren Extremität ausgebildet, insbesondere als knieübergreifende Orthese, als Knie-Knöchel-Fußorthese, als Hüft-Knie-Knöchel-Fußorthese oder als Knöchel-Fußorthese, da bei Orthesen der unteren Extremität vergleichsweise hohe Kräfte und Momente aufgenommen und übertragen werden müssen, die über die zweite Gelenkeinrichtung gut aufgenommen werden können. Dazu ist es vorteilhaft, wenn die Stützelemente kraftübertragend und momentenübertragend an dem Oberteil und dem Unterteil festgelegt sind. Insbesondere sind die Stützelemente mit dem Oberteil und dem Unterteil verschraubt, gegebenenfalls in vorgeformte Vorsprünge oder Ausnehmungen eingesetzt, um eine feste Zuordnung und somit eine Kraft- und Momentenübertragung zu gewährleisten. Die Stützelemente können aus Metall oder einem faserverstärkten Kunststoff ausgebildet sein, um eine ausreichende Festigkeit und Stabilität zur Übertragung der in der Orthese wirkenden Kräfte und Momente zu gewährleisten.

In einer Weiterbildung der Erfindung ist zumindest eines der Stützelemente als ein Funktionselement ausgebildet oder an zumindest einem Stützelement ist ein solches Funktionselement befestigt. Das Funktionselement kann als Antrieb, Dämpfer, Bremser, Verriegelungseinrichtung, Messwertaufnehmer und/oder Energiespeicher ausgebildet sein. Der Antrieb kann als elektromotorischer Antrieb oder hydraulischer Antrieb ausgebildet sein, wobei der hydraulische Antrieb mit einer Pumpe und/oder einem Energiespeicher gekoppelt sein kann, beispielsweise einer mechanischen oder pneumatischen Feder. Über den Antrieb oder den Energiespeicher ist ein Hydraulikvolumen mit Druck beaufschlagbar, um einen Kolben, beispielsweise einen Linearkolben oder einen Schwenkkolben, zu verlagern um eine Orthesenkomponente gegenüber der anderen Orthesenkomponente zu verlagern. Dadurch ist es möglich, ein Oberteil der Gelenkeinrichtung relativ zu dem Unterteil zu verschwenken, um eine Flexion und/oder Extension zu bewirken oder zumindest zu unterstützen. Darüber hinaus ist es möglich, das Funktionselement als eine Dämpfereinrichtung auszugestalten, insbesondere als ein Hydraulikdämpfer, bei dem über die Veränderung eines hydraulischen Widerstandes die Verschwenkbewegung in Extensionsrichtung und/oder Flexionsrichtung beeinflusst werden kann. Eine Bremse oder ein verstellbarer Endanschlag kann ebenfalls als Funktionselement ausgebildet oder Teil des Funktionselementes sein. Darüber hinaus kann das Funktionselement als Messwertaufnehmer ausgebildet sein oder einen solchen aufweisen, um Bewegungsdaten und/oder Belastungsdaten des Oberteils, des Unterteils oder weitere Komponenten der Orthese oder eines Orthesensystems zu erfassen. Die Messwertaufnehmer können als Kraftsensoren, Winkelsensoren, Wegsensoren, Momentsensoren oder Lagesensoren ausgebildet sein. Darüber hinaus können die Messwertaufnehmer Bewegungsdaten wie Beschleunigungen oder Geschwindigkeiten aufnehmen, um diese über eine Steuereinrichtung zur Veränderung von Widerständen oder Einstellungen an einem Funktionselement, beispielsweise einer Dämpfereinrichtung oder einem Antrieb vorzunehmen. In Abhängigkeit von Werten, die über die Messwertaufnehmer erfasst werden, kann dann die Orthese gesteuert werden, um an den Nutzer oder an die jeweilige Bewegung oder Bewegungssituation angepasste Einstellungen vornehmen zu können. Ein Energiespeicher als Funktionselement ist insbesondere als Feder ausgebildet, sodass beispielsweise Bewegungsenergie in Verformungsenergie beim Abbremsen einer Bewegung umgewandelt und gespeichert und zu einem anderen Zeitpunkt wieder kontrolliert abgegeben werden kann. Dadurch entfällt die Umwandlung von Bewegungsenergie in elektrische Energie und Speicherung elektrischer Energie in einem Akkumulator. Neben einer integralen Ausgestaltung des Stützelementes als Funktionselement ist es möglich, dass ein solches Funktionselement an dem zumindest einen Stützelement befestigt ist, um neben einer Führung der Gliedmaße die jeweilige Bewegung analysieren und/oder beeinflussen zu können.

Das erfindungsgemäße System aus einer Orthese, wie sie voranstehend beschrieben worden ist, und einer Vielzahl unterschiedlicher Funktionselemente, beispielsweise unterschiedlichen Antrieben, unterschiedlichen Dämpfern, Bremseinrichtungen, Messwertaufnehmern und/oder Federn sieht vor, dass diese unterschiedlichen Funktionselemente an denselben Befestigungseinrichtungen über zumindest eine Lagerplatte an dem Oberteil und/oder Unterteil festlegbar sind. Neben unterschiedlichen Funktionselementen, die voneinander abweichende Funktionen ausführen, können auch Varianten von Funktionselementen mit gleichen Funktionen montiert, getestet und angepasst werden. Über die Befestigungseinrichtungen an der Lagerplatte können die unterschiedlichen Funktionselemente einfach ausgetauscht werden, sodass es möglich ist, über die modulare Ausgestaltung der Funktionselemente und der standardisierten Anordnung und Orientierung der Befestigungseinrichtungen der Funktionselemente zu den Befestigungseinrichtungen an der Lagerplatte eine leichte Austauschbarkeit und damit eine leichte Anpassbarkeit an den jeweiligen Patienten oder die jeweilige Nutzungsabsicht zu erreichen. Die Funktionselemente können in Abhängigkeit von dem Patienten, dem Einsatzzweck und/oder anderen Rahmenbedingungen der Versorgung ausgewählt und an eine standardisierte Orthese angepasst werden. Über die Funktionselemente werden dann eine Individualisierung und eine Anpassung der Orthese an den jeweiligen Nutzer vorgenommen. Dazu ist es vorgesehen, dass an jedem Funktionselement, das Teil des Systems ist, Befestigungseinrichtungen oder Befestigungselemente angeordnet oder ausgebildet sind, die korrespondierend zu den dafür vorgesehenen Befestigungseinrichtungen oder Aufnahmeeinrichtungen an der Lagerplatte für das Oberteil und/oder Unterteil positioniert sind. Dadurch kann beispielsweise durch Austausch eines Funktionselementes, das als passiver Hydraulikdämpfer ausgebildet ist, gegen ein Funktionselement, das als Hydraulikantrieb ausgebildet ist, aus einer passiven Orthese eine aktive Orthese werden, über die Bewegungen eingeleitet, ausgeführt oder unterstützt werden können. Ebenfalls ist es möglich, statt einem Funktionselement, das die Bewegung der Orthese beeinflusst, eine reine Erfassungseinheit anzuordnen, über die Bewegungsdaten erfasst werden, beispielsweise um einen Heilungsfortschritt oder den Verlauf einer Beeinträchtigung dokumentieren zu können.

Eine Weiterbildung des erfindungsgemäßen Systems sieht vor, dass alle Funktionselemente Befestigungselemente aufweisen, die korrespondierend zu Aufnahmeeinrichtungen für die Befestigungselemente zur Festlegung an dem Oberteil und/oder dem Unterteil über die Lagerplatte angeordnet sind. Die korrespondierende Anordnung der Befestigungselemente zu den Aufnahmeeinrichtungen für die Befestigungselemente erlaubt insbesondere die Anordnung von zumindest einem Distanzelement zwischen der eigentlichen Orthese und dem Funktionselement, um zu gewährleisten, dass die individuelle Anpassbarkeit an den jeweiligen Patienten gewährleistet wird. So können bei unteren Extremitäten beispielsweise eine X-Bein-Stellung eine andere Beabstandung eines Funktionselementes von einem Oberteil oder einem Unterteil erfordern als eine O-Bein-Stellung, sodass Distanzelemente zwischen dem Oberteil und/oder Unterteil oder der Lagerplatte sowie dem Funktionselement angeordnet sein können, insbesondere wenn das Funktionselement eine besondere Ausrichtung aufweisen oder zu einen anderen Komponente besonders ausgerichtet sein müssen.

Das Funktionselement in dem System ist bevorzugt auswechselbar an der Lagerplatte und ggf. dem Oberteil und dem Unterteil festgelegt, um eine Anpassbarkeit an den jeweiligen Einsatzzweck oder Patienten zu erleichtern. Das obere Stützelement und das untere Stützelement können zu einem Modul zusammengefasst montiert sein, sodass ein modularer Aufbau und damit ein leichter Austausch und eine leichte Anpassbarkeit erfolgen können.

Das Verfahren zum Anpassen einer Orthese, wie sie oben beschrieben worden ist, an einem Patienten, wobei die Orthese über Einrichtungen zur Aufnahme oder Festlegung eines Körperteils oder einer Gliedmaße an dem Körperteil oder der Gliedmaße befestigt wird sieht vor, dass, nachdem ggf. eine erste Gelenkeinrichtung aus einer Vielzahl an verschiedenen ersten Gelenkeinrichtungen ausgewählt worden ist und diese erste Gelenkeinrichtung mit Einrichtungen zur Aufnahme oder zur Festlegung eines Körperteils oder einer Gliedmaße verbunden worden sind, eine zweite Gelenkeinrichtung aus einer Vielzahl an verschiedenen Gelenkeinrichtungen ausgewählt und dann ein oberes und unteres Stützelement, die über die ausgewählte zweite Gelenkeinrichtung miteinander verbunden sind, mit dem Oberteil und mit dem Unterteil der ersten Gelenkeinrichtung verbunden, um so basierend auf einer Auswahl verschiedener Gelenke oder Gelenkeinrichtungen und ein jeweiliges Permutieren der Gelenkeinrichtungen eine große Variation unterschiedlicher Orthesentypen bereitstellen und an den jeweiligen Patienten anpassen zu können. Der modulare Aufbau der einzelnen Komponenten, insbesondere der ersten Gelenkeinrichtungen und der zweiten Gelenkeinrichtungen, ermöglicht einen leichten Austausch untereinander. In einer Weiterbildung der Erfindung ist es vorgesehen, dass zunächst aus einer Vielzahl erster Gelenkeinrichtungen die geeignete Gelenkeinrichtung für den Patienten ausgewählt wird und mit den Schienen und Befestigungseinrichtungen ausgestattet wird. Nach der Auswahl einer ersten Gelenkeinrichtung, die als am besten geeignet angesehen wird, kann eine Vielzahl zweiter Gelenkeinrichtungen an der ersten Gelenkeinrichtung über die Stützelemente befestigt und ausprobiert oder ausgetauscht werden, sodass eine Individualisierung oder eine Anpassung an den jeweiligen Patienten, den Einsatzzweck oder einen sich verändernden Zustand eines Patienten erfolgen kann. Für das Grundgelenk, also die erste Gelenkeinrichtung, werden insbesondere Lasten in medial-lateral-Richtung aufgenommen, während über die zweite Gelenkeinrichtung Lasten in anterior-posterior-Richtung aufgenommen und abgeleitet werden können. Das Grundgelenk ist insbesondere als ein Freiläufergelenk ausgebildet, während die zweite Gelenkeinrichtung insbesondere mit Funktionselementen wie Antrieben, Dämpfern, Messwertaufnehmer, Freigabe- oder Verriegelungseinrichtungen, Vorbringer, Federn oder anderer Energiespeicher ausgerüstet sein kann.

Nachfolgend werden Ausführungsbeispiele anhand der beigefügten Figuren näher erläutert. Es zeigen:
- Figuren 1 - 3 -: eine KAFO in Front-Medial-und Lateralansicht;
- Figuren 4 - 8 -: eine erste Variante der Erfindung in Schnittansicht, Frontalansicht, Lateralansicht, Rückansicht und Medialansicht;
- Figuren 9 - 13 -: eine zweite Variante in Schnittansicht, Frontalansicht, Lateralansicht, Rückansicht und Medialansicht;
- Figuren 14 - 18 -: eine dritte Variante in Schnittansicht, Frontalansicht, Lateralansicht, Rückansicht und Medialansicht;
- Figuren 19 - 21 -: Seitenansichten eines Orthesensystems; sowie
- Figuren 19a - 21a -: mediale Seitenansichten der Figuren 19 bis 21.

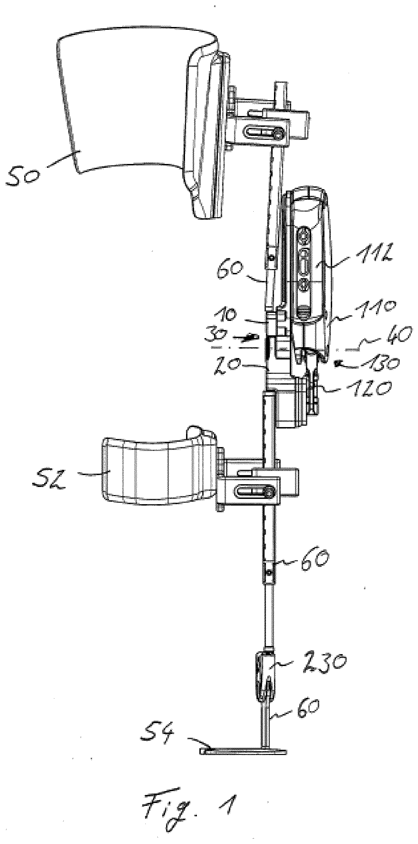

Figur 1 zeigt in einer Frontalansicht eine sogenannte KAFO (knee-ankle-foot ortthosis) zur Anlage an einem Bein. Die Orthese wird über Aufnahmeeinrichtungen 50, 52 und 54 an dem Bein festgelegt, gegebenenfalls zusammen mit zusätzlichen Gurten, die nicht dargestellt sind. Der Oberschenkel wird in einer Oberschenkelschale 50 aufgenommen, der Unterschenkel in einer Unterschenkelschale 52 und der Fuß wird auf einer Fußplatte 54 aufgestellt. Die Einrichtungen 50, 52, 54 zur Aufnahme von Körperteilen sind an Schienen 60 befestigt, die entlang der jeweiligen Gliedmaße verlaufen, im dargestellten Ausführungsbeispiel lateral zu dem nicht dargestellten Bein. Von der Oberschenkelschale 50 erstreckt sich eine Oberschenkelschiene 60 bis zu einem Oberteil 10 und wird dort formschlüssig in einem sogenannten Schienenkasten über Schrauben festgelegt. Das Oberteil 10 bildet somit das distale Endstück der Oberschenkelschiene 60. An dem Oberteil 10 ist um eine Schwenkachse 40 ein Unterteil 20 befestigt, so dass das Oberteil 10 zusammen mit dem Unterteil 20 eine Gelenkeinrichtung 30 ausbildet, die auf der Höhe eines natürlichen Kniegelenkes angeordnet ist. An dem Unterteil 20 ist eine Unterschenkelschiene 60 befestigt, das Unterteil 20 bildet somit den proximalen Abschluss der Unterschenkelschiene 60 aus. An dem distalen Ende der Unterschenkelschiene 60 ist ein Knöchelgelenk 230 auf der Höhe eines natürlichen Knöchelgelenkes angeordnet, an das sich eine Schienenkomponente 60 anschließt, die die Fußplatte 54 mit dem Orthesenknöchelgelenk 230 verbindet. Die Fußplatte 54 ist über das Orthesenknöchelgelenk 230 schwenkbar mit der Unterschenkelschiene 60 verbunden.

An dem Orthesenkniegelenk mit der Gelenkeinrichtung 30 sind lateral zu den Schienen 60 Stützelemente 110, 120 befestigt. Ein oberes Stützelement 110 ist an dem Oberteil 10 und gegebenenfalls an der Oberschenkelschiene 60 momentenübertragend und kraftübertragend befestigt. Entsprechend ist ein unteres Stützelement 120 an dem Unterteil 20 kraftübertragend und momentübertragend befestigt, insbesondere angeschraubt oder über Formschusselemente daran festgelegt. Das obere Stützelement 110 ist mit dem unteren Stützelement 120 schwenkbar unter Ausbildung einer zweiten Gelenkeinrichtung 130 schwenkbar verbunden, so dass lateral beabstandet zu der ersten Gelenkeinrichtung 30 zwischen dem Oberteil 10 und dem Unterteil 20 eine zweite, parallele und koaxial um die Schwenkachse 40 verschwenkbare zweite Gelenkeinrichtung 130 im Bereich des Orthesenkniegelenkes ausgebildet wird. Eine korrespondierende Ausgestaltung kann an einem Orthesenknöchelgelenk oder auch an einem Orthesenhüftgelenk ausgebildet sein.

Die beiden Stützelemente 110, 120 können insbesondere lösbar an dem Oberteil 10 und Unterteil 20 befestigt sein, um eine leichte Anpassung einer Basisorthese mit einer alternativ ausgebildeten, lateral angeordneten zweiten Gelenkeinrichtung 120 auszubilden.

In dem oberen Stützelement 110 ist eine Aufnahme 112 für weitere Komponenten vorgesehen, beispielsweise Antriebe, Dämpfereinrichtungen, Steuerungseinrichtungen, Sensoren oder dergleichen.

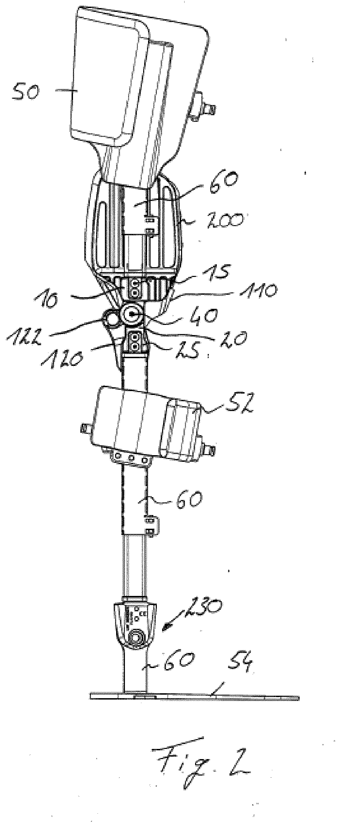

Figur 2 zeigt die KAFO gemäß Figur 1 in einer Medialansicht. An dem Oberteil 10 ist ein Schienenkasten 15 mit Schrauben ausgebildet, in dem die Oberschenkelschiene 60 eingesetzt und formschlüssig festgelegt ist. An der Oberschenkelschiene 60 ist die Oberschenkelschale 50 befestigt. Distal zu dem Oberteil 10 ist das Unterteil 20 schwenkbar um die Schwenkachse 40 festgelegt. Die Unterschenkelschiene 60 ist in dem Schienenkasten 25 mit dem Unterteil 20 verschraubt. In der Medialansicht ist die Aufnahme 122 in Gestalt einer Durchgangsöffnung zur Aufnahme beispielsweise eines Bolzens zur Festlegung einer Kolbenstange zu erkennen, die Aufnahmeeinrichtung in dem oberen Stützelement 110 ist nicht zu erkennen, das untere Stützelement 120 ist nur teilweise zu erkennen. An dem Oberteil 10 ist eine Lagerplatte 200 angeordnet, die sich in Proximalrichtung über das Oberteil 10 hinaus erstreckt und wesentlich breiter als das Oberteil 10 ist. An der Lagerplatte 200 ist das obere Stützelement 110 festgelegt, die Befestigung des oberen Stützelementes 110 an der Lagerplatte 200 wird später detailliert beschrieben werden. Das untere Stützelement 120 ist direkt an dem Unterteil 20 lösbar befestigt.

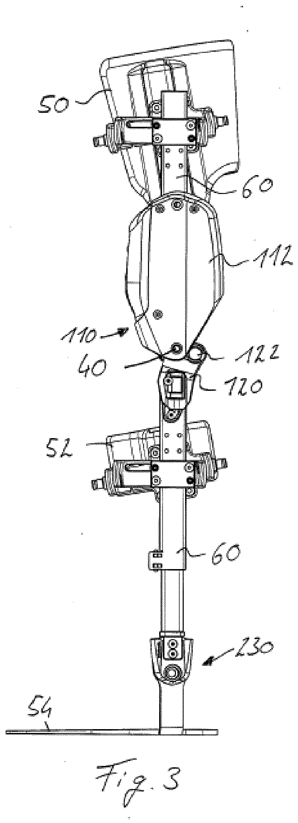

Figur 3 zeigt die Orthese gemäß Figur 1 in einer Lateralansicht, in der sowohl das obere Stützelement 110 als auch das untere Stützelement 120 besser zu erkennen sind. Die Aufnahmeeinrichtung 112 in dem oberen Stützelement 110 und die Aufnahmeeinrichtung, 122 dem unteren Stützelement 120 sind ebenso zu erkennen wie die schwenkbare Ausgestaltung der zweiten Gelenkeinrichtung 130 und die koaxiale Ausgestaltung der Schwenkachse 40 der zweiten Gelenkeinrichtung 130 zu der Schwenkachse der ersten Gelenkeinrichtung 30 des Oberteils 10 und des Unterteils 20. Die Aufnahmeeinrichtung 112, die ein Gehäuse sein kann, verdeckt die Lagerplatte 200, die die zwischen dem Oberteil 10 und dem oberen Stützelement 110 befindet, vollständig.

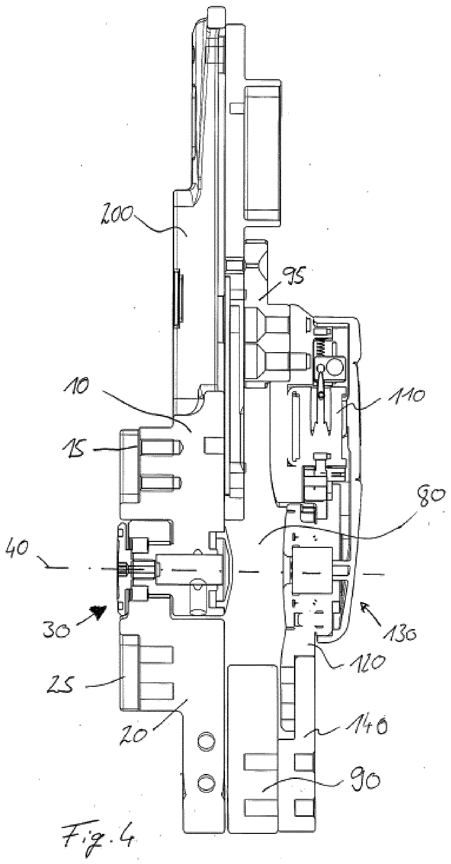

Die Figuren 4 bis 8 zeigen Einzeldarstellungen der Ausgestaltung der Gelenkeinrichtungen ohne die Schienen 60 und die Einrichtungen 50, 52, 54 zur Aufnahme des Oberschenkels, Unterschenkels und des Fußes in einer frontalen Schnittansicht, in einer Vorderansicht, in einer Lateralansicht, in einer rückwärtigen Ansicht sowie in einer Medialansicht.

Figur 4 zeigt eine Teilansicht einer Orthese in Schnittdarstellung mit dem Oberteil 10, dem Unterteil 20 und den jeweils daran ausgebildeten Befestigungseinrichtungen 15, 25 zur lösbaren Befestigung der Schienen. In den Befestigungseinrichtungen 15, 25 werden die Schienen eingelegt und über Schrauben daran befestigt. Die Schrauben werden in Gewindebohrungen innerhalb des Oberteils 10 und des Unterteils 20 eingeschraubt.

Das Oberteil 10 und das Unterteil 20 sind um eine Schwenkachse 40 unter Ausbildung einer Gelenkeinrichtung 30 miteinander verbunden. Lateral zu den nicht dargestellten Schienen und dem Oberteil 10 und dem Unterteil 20 sind an dem Oberteil 10 und dem Unterteil 20 zwei Stützelemente 110, 120 befestigt, im dargestellten Ausführungsbeispiel angeschraubt. Ein oberes Stützelement 110 ist an dem Oberteil 10 über die Lagerplatte 200 über Schrauben kraftübertragend und momentenübertragend befestigt. Die Lagerplatte 200 ist als separates Bauteil ausgebildet und an dem Oberteil lösbar befestigt. Korrespondierend ist ein unteres Stützelement 120 kraftübertragend und momentenübertragend an dem Unterteil 20 befestigt, hier unter Zwischenschaltung eines Distanzstückes 90. Beide Stützelemente 110, 120 sind schwenkbar miteinander verbunden und bilden eine zweite Gelenkeinrichtung 130 aus, deren Schwenkachse koaxial zu der Schwenkachse der ersten Gelenkeinrichtung 30 verläuft. Dadurch ist es möglich, dass ohne Verdrehungen oder Zwängungen eine Verschwenkung der Orthesenkomponenten um die Schwenkachse 40 erfolgen kann.

Die beiden Gelenkeinrichtungen 30, 130 sind lateral zueinander beabstandet angeordnet, so dass ein Freiraum 80 zwischen den Gelenkeinrichtungen 30, 130 ausgebildet wird. Durch die Doppelung des Gelenkes in dem Bereich der Schienenaufnahmen ist es möglich, in hochbelasteten Bereichen einer Orthese eine wirksame Aufnahme und Weiterleitung von Kräften und Momenten zu bewirken. Die in der Orthese auftretenden Kräfte werden durch den rahmenartigen Aufbau in dem Orthesengelenk mit den beiden Gelenkeinrichtungen 30, 130 vorteilhaft aufgenommen und weitergeleitet. Die Kraftdurchleitlinien in Längserstreckung, also von proximal nach distal in der gestreckten Stellung, werden aufgeteilt und verlaufen sowohl durch das Oberteil 10 und das Unterteil 20 mit der zwischen diesen beiden Strukturbauteilen ausgebildeten ersten Gelenkeinrichtung 30 und dem oberen Stützelement 110, dem unteren Stützelement 120 und der dazwischen ausgebildeten zweiten Gelenkeinrichtung 130. Im Bereich der Ankopplung der Stützelemente 110, 120 an das Ober- und Unterteil 10, 20 werden die Kräfte und Momente aufgeteilt bzw. zusammengeführt, so dass sich ein geschlossener Kraftzug ergibt. Die lateral angeordnete Gelenkeinrichtung 30 arbeitet somit als Mitläufergelenk. Der Abstand zwischen den beiden Gelenkeinrichtungen 30, 130 entspricht der doppelten Dicke der Schienen. Je größer der Abstand der Gelenkeinrichtungen 30, 130 voneinander ist, desto stabiler ist das gesamte Orthesengelenk, allerdings führt ein sehr großer Abstand Nachteilen bei dem Tragekomfort.

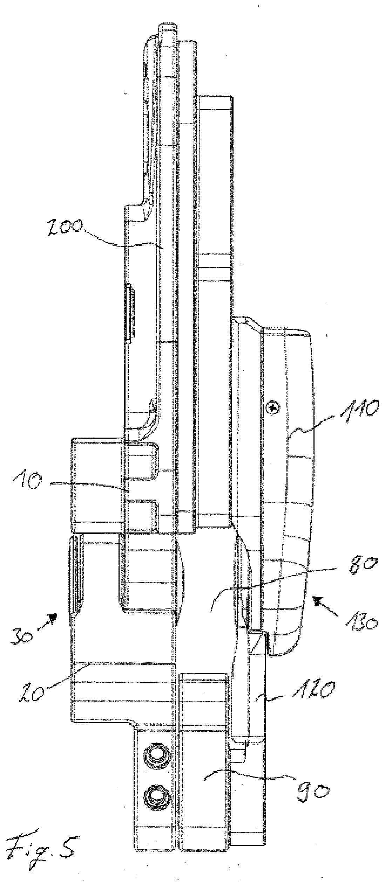

Figur 5 zeigt den Ausschnitt der Orthese gemäß Figur 4 in einer Draufsicht von vorn. Sowohl das Oberteil 10 als auch das Unterteil 20 sowie das obere und untere Stützelement 110, 120 sind mit den lateral zueinander beabstandeten, also entlang der Schwenkachse 40 zueinander beabstandeten Gelenkeinrichtungen 30, 130 zu erkennen. Zwischen den Gelenkeinrichtungen 30, 130 ist der Freiraum 80 zu erkennen. Um den Freiraum 80 und den Abstand zwischen den Gelenkeinrichtungen 30, 130 trotz einer flachen Bauweise des unteren Stützelementes 120 realisieren zu können, ist ein Distanzstück 90 zwischen dem Unterteil 20 und dem unteren Stützelement 120 angeordnet, das dem Freiraum 80 zwischen den beiden Gelenkeinrichtungen 30, 130 entspricht. Statt eines separaten Distanzelementes 90 kann über einen entsprechenden Absatz, der an dem unterem Stützelement 120 angeformt ist, ein Freiraum 80 realisiert werden. An dem oberen Stützelement 110 wird der Distanzausgleich über das Distanzelement 95, das in der Figur 4 dargestellt ist, zu der Lagerplatte 200 hergestellt.

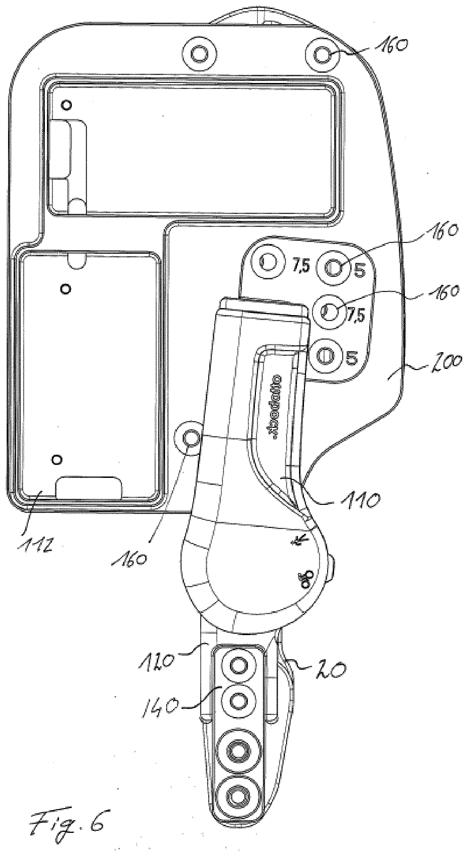

Figur 6 zeigt die Ausgestaltung gemäß Figuren 4 und 5 in einer Außenansicht, also in einer lateralen Draufsicht. Neben dem oberen Stützelement 110 hinter einer Verkleidung und dem unteren Stützelement 120 ist das Unterteil 20 im Hintergrund nur teilweise zu erkennen. An dem oberen Stützelement 110 sind Aufnahmen 112 für weitere Komponenten, beispielsweise Steuerungseinrichtungen, Sensoren oder Aktuatoren ausgebildet. An dem unteren Stützelement 120 ist eine Art Schienenkasten ausgebildet, ähnlich dem in dem Unterteil 20. In dem Schienenkasten ist eine Befestigungsstrebe 140 eingesetzt und über Schrauben mit dem unteren Stützelement 120 verbunden. Die Strebe 140 wird über weitere Schrauben über das Distanzstück 90 mit dem Unterteil 20 kraftübertragend und momentenübertragend verbunden. Durch die Anbindung des unteren Stützelementes 120 an das Unterteil 20 über eine Strebe 140 sowie ein Distanzelement 90 kann eine leichte Anpassbarkeit an unterschiedliche Einsatzbedingungen oder unterschiedliche Orthesen mit einem Oberteil 10 und einem Unterteil 20 erreicht werden. An dem nicht zu erkennenden Oberteil 10 ist die Lagerplatte 200 angeordnet, an der wiederum das obere Stützelement 110 befestigt ist. An der Lagerplatte 200 sind eine Vielzahl von Befestigungseinrichtungen 160 angeordnet oder ausgebildet, über die es möglich ist, das obere Stützelement 110 in unterschiedlichen Orientierungen und an unterschiedlichen Positionen an der Lagerplatte 200 zu befestigen.

Neben einer Positionierung des oberen Stützelementes 110 an unterschiedlichen Stellen ist es möglich, unterschiedliche obere Stützelemente 110 an der Orthese zu befestigen, um eine zweite, parallele Gelenkeinrichtung 130 auszubilden und Variationen in der Konstruktion leicht auszuprobieren oder auch dauerhaft zu etablieren. Darüber hinaus ist über die Lagerplatte 200 und die Vielzahl von Befestigungseinrichtungen 160 die Möglichkeit gegeben, Zusatzkomponenten wie Energiespeicher, Steuerungseinrichtungen, Bedienelemente und dergleichen an der Orthese anzuordnen und mit dem Oberteil 10 zu koppeln. Ist die Lagerplatte 200 an dem Unterteil 20 angeordnet, kann eine entsprechende Anordnung von Zusatzkomponenten an dem Unterteil 20 erfolgen.

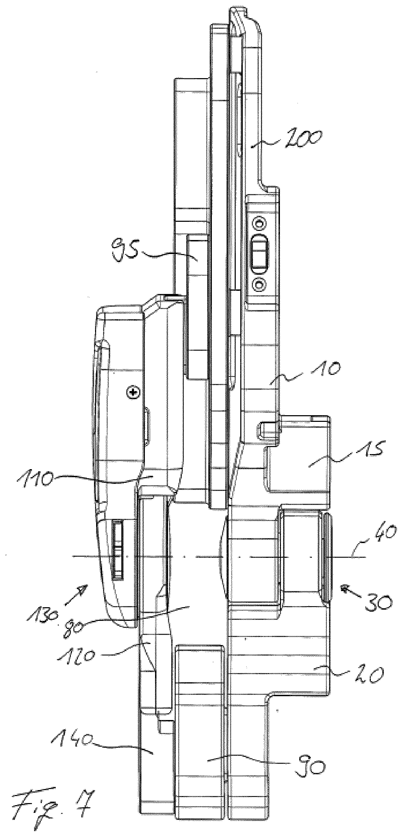

Figur 7 zeigt die Orthesenkomponente in Rückansicht. Die Distanzstücke 90, 95 sind ebenso zu erkennen wie die Ober- und Unterteile 10, 20, die Gelenkeinrichtungen 30, 130 sowie die oberen und unteren Stützelemente 110, 120. Die Befestigungsschiene 140 und die Aufnahme 15 für eine Oberschenkelschiene sind ebenso dargestellt wie die koaxiale Schwenkachse 40 für die beiden Gelenkeinrichtungen 30, 130.

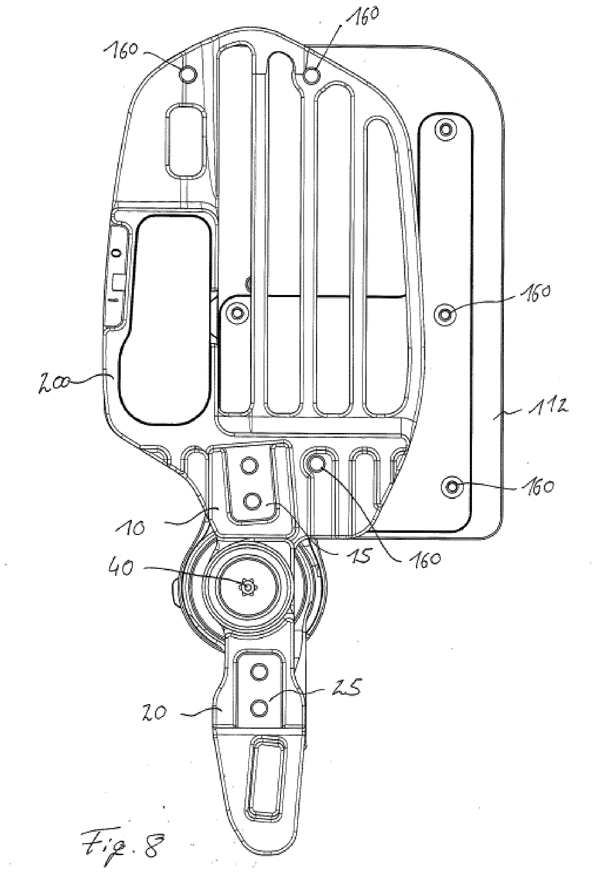

Figur 8 zeigt die Ausführungsformen gemäß der Figuren 4 bis 7 in einer Medialansicht, in der die Befestigungseinrichtungen 15, 25 in Gestalt von Schienenkästen für die nicht dargestellten Schienen 60 sowie die senkrecht zu der Blattebene verlaufende Schwenkachse 40 dargestellt sind, im Hintergrund ist die Aufnahmeeinrichtung 112 des oberen Stützelementes 110 zu erkennen. In der Medialansicht ist die Lagerplatte 200 mit der Vielzahl an Befestigungseinrichtungen 160 zu erkennen, die an dem Oberteil 10 befestigt ist. An der Lagerplatte 200, die mit Durchbrüchen versehen ist, beispielsweise um verschiebliche Befestigungselemente anzuordnen oder um weitere Komponenten an der Lagerplatte festlegen zu können, ist eine Vielzahl von Befestigungseinrichtungen 160 ausgebildet, über die an unterschiedlichen Stellen unterschiedliche zweite Gelenkeinrichtungen 130 festlegbar sind.

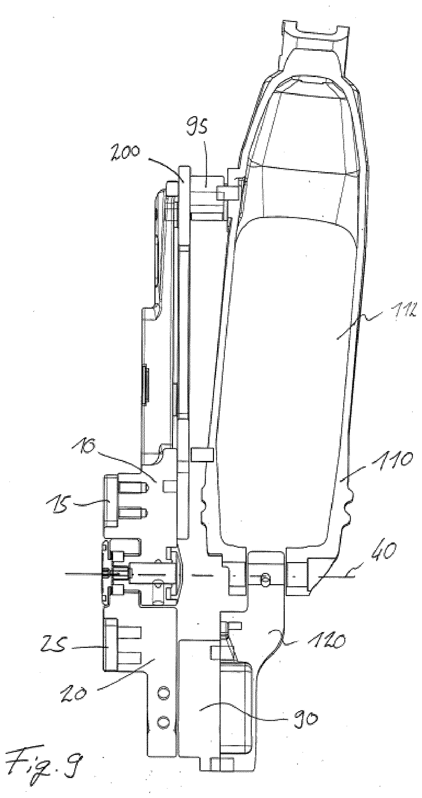

Eine Variante der Erfindung ist in den Figuren 9 bis 13 dargestellt. Der grundsätzliche Aufbau entspricht der der Figuren 4 bis 8, so dass hier nur auf Abweichungen eingegangen wird. Anders als bei der Ausführungsform gemäß der Figuren 4 bis 8 ist das obere Stützelement 110 nicht im Wesentlichen parallel orientiert zu dem Oberteil 10 angeordnet, sondern weist eine leichte Neigung nach lateral auf, was durch das proximale Distanzelement 95 zwischen der Aufnahme 112 und der Lagerplatte 200 ausgeglichen wird. Die Neigung in Medial-Lateralrichtung kann eingestellt werden, dazu kann die Gelenkeinrichtung 130 einstellbar ausgebildet sein, beispielsweise über ein Schwenkgelenk, das eine Medial-Lateral-Verschwenkung zulässt, ohne die Orientierung der Schwenkachse 40 der zweiten Gelenkeinrichtung 130 zu verändern. Dazu wäre beispielsweise proximal zu der Schwenkachse 40 ein Gelenk vorzusehen, das in der gestreckten Stellung der Orthese eine Schwenkachse aufweist, die in die übliche Gehrichtung zeigt, also senkrecht zu der Blattebene von anterior nach posterior verläuft. Mit einer solchen Winkeleinstellbarkeit, bei der die Abstützung über das Distanzelement 95 erfolgt oder auch über ein Gewinde einstellbar ist, kann eine Anpassung an die jeweils in der Aufnahmeeinrichtung 112 angeordneten Komponenten, wie Antriebe, Dämpfer oder Steuerungseinrichtungen erfolgen. In der Schnittdarstellung gemäß Figur 9 ist die Aufnahme 112 als Hohlraum ausgebildet, in dem beispielsweise ein hydraulischer Dämpfer oder ein elektromotorischer Antrieb einsetzbar und darin festlegbar ist.

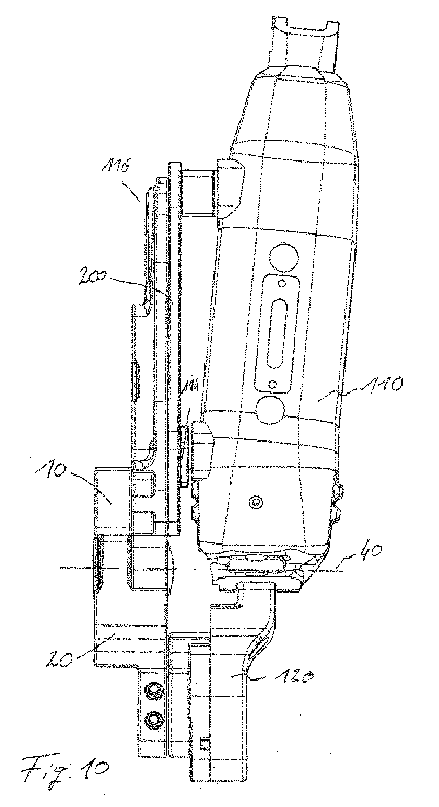

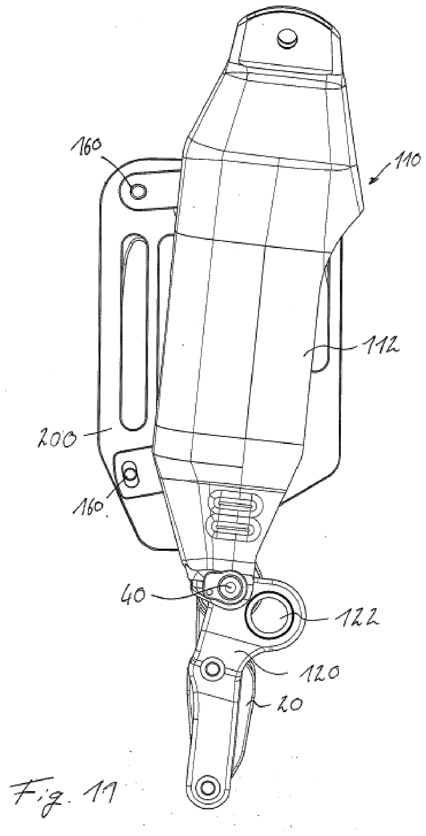

Figur 10 zeigt die Ausgestaltung des oberen Stützelementes als Gehäuse, in dem die Aufnahme 112 ausgebildet ist. Das obere Stützelement 110 ist an zwei Befestigungsstellen 114, 116 mit dem Oberteil 10 über die Lagerplatte 200 und an dieser über Schrauben oder Bolzen kraftübertragend und auswechselbar gekoppelt. In der Figur 11 in der Lateralansicht sind die Aufnahme 112 am oberen Stützelement 110 und die Aufnahme 122 an dem unteren Stützelement 120 zu erkennen. Das untere Stützelement 120 weist keinen Schienenkasten wie in dem Ausführungsbeispiel der Figuren 4 bis 8 auf, sondern wird unmittelbar über das Distanzelement 90 an dem Unterteil 20 angeschraubt. Die Aufnahme 122 an dem unteren Stützelement 120 ist eine ringförmige Lageraufnahme, in der beispielsweise ein Bolzen einer Kolbenstange für einen Antrieb oder einen Dämpfer festlegbar ist, um eine Verschwenkung des Unterteils 20 zu dem Oberteil 10 um die Schwenkachse 40 zu bewirken oder abzudämpfen.

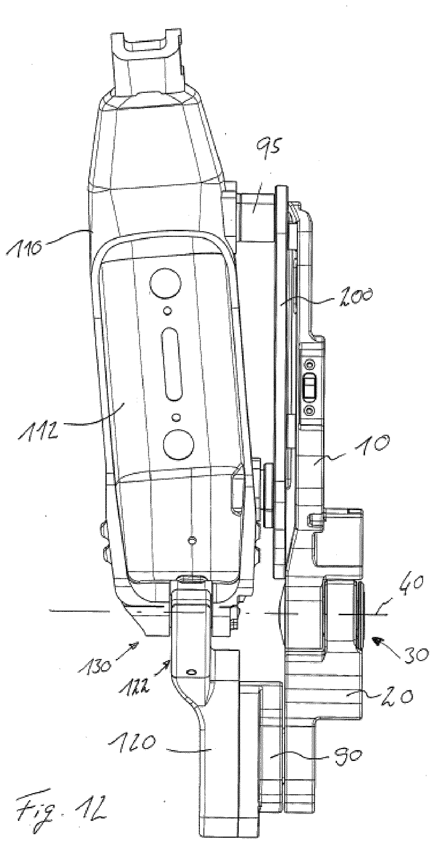

In der rückwärtigen Ansicht gemäß Figur 12 ist die hohle Ausgestaltung des oberen Stützelementes 110 mit der Aufnahme 112 ebenso zu erkennen wie die ringförmige Anformung der unteren Aufnahme 122.

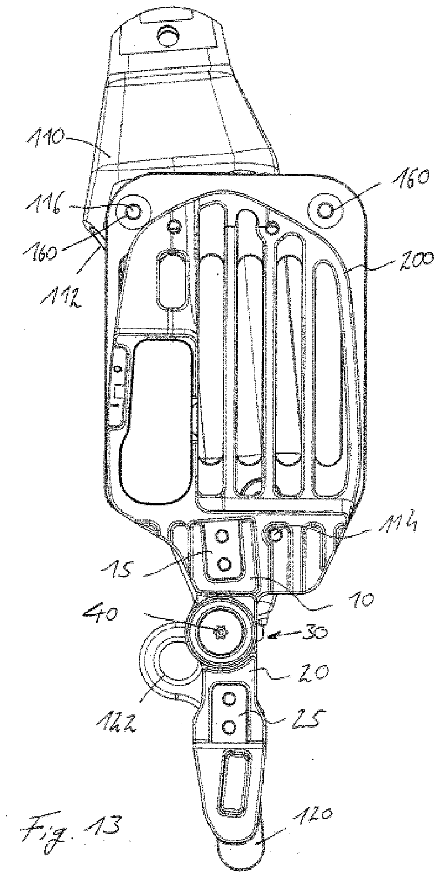

Figur 13 zeigt eine Medialansicht des Orthesenkniegelenkes mit Oberteil 10 und Schienenkasten 15, Unterteil 20 mit Schienenkasten 25 und der ersten Gelenkeinrichtung 30 zur Verschwenkung um die Schwenkachse 40. An der Lagerplatte 200 sind die Befestigungsstellen 114, 116 und Befestigungseinrichtungen 160 für die Festlegung des oberen Stützelementes 110 zu erkennen.

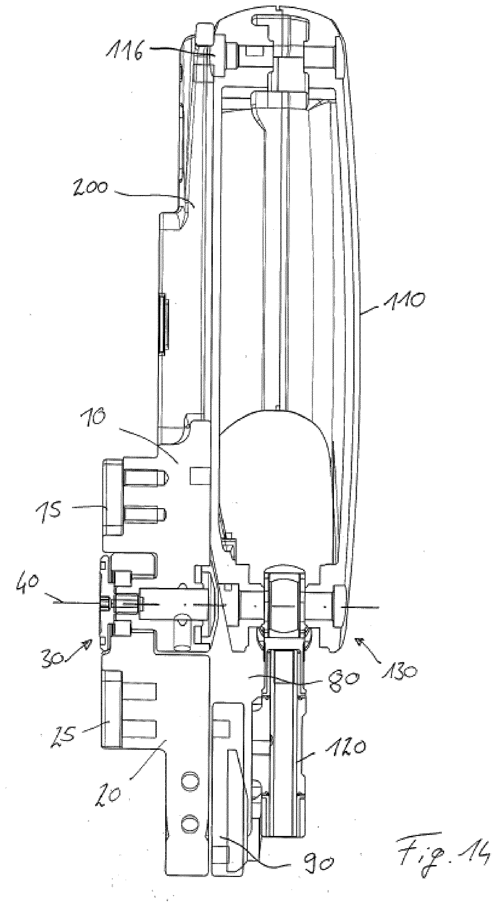

Eine dritte Variante der Erfindung ist in den Figuren 14 bis 18 dargestellt. Der Aufbau des Orthesengelenkes, das als Hüftgelenk, Kniegelenk oder Knöchelgelenk oder aber auch als Ellenbogengelenk ausgebildet sein kann, entspricht grundsätzlich dem der Variante der Figuren 9 bis 13, jedoch mit einem im Wesentlichen parallel zu dem Oberteil 10 und der Lagerplatte 200 orientierten oberen Stützelement 110. Aufgrund der gehäuseartigen Ausgestaltung des oberen Stützelementes 110 ist eine Anordnung eines oberen Distanzelementes nicht notwendig. Weiterhin ist ein Freiraum 80 zwischen den beiden Gelenkeinrichtungen 30, 130 ausgebildet, so dass die Kraftdurchleitlinien durch die erste Gelenkeinrichtung 30 und die zweite Gelenkeinrichtung 130 beabstandet zueinander in Längserstreckung der Orthese verlaufen können. Der obere Befestigungspunkt 116 ist relativ weit von der Schwenkachse 40 entfernt, um eine hohe Stabilität in der Gesamtkonstruktion zu erreichen. Zwischen dem unteren Stützelement 120 und dem Unterteil 20 ist wiederum ein Distanzelement 90 angeordnet.

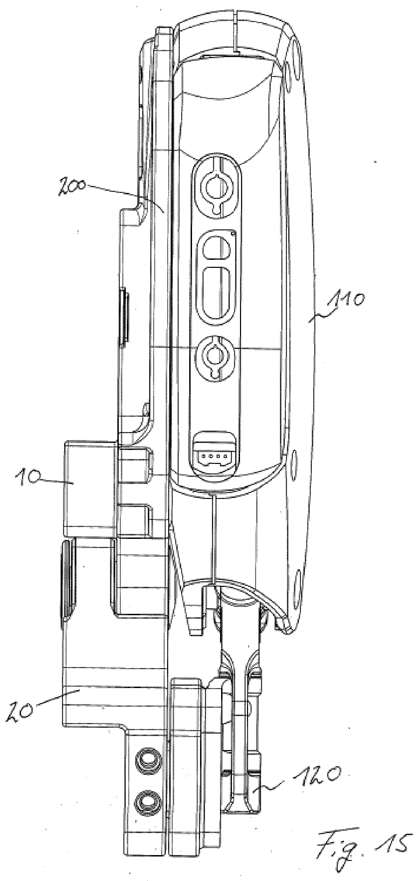

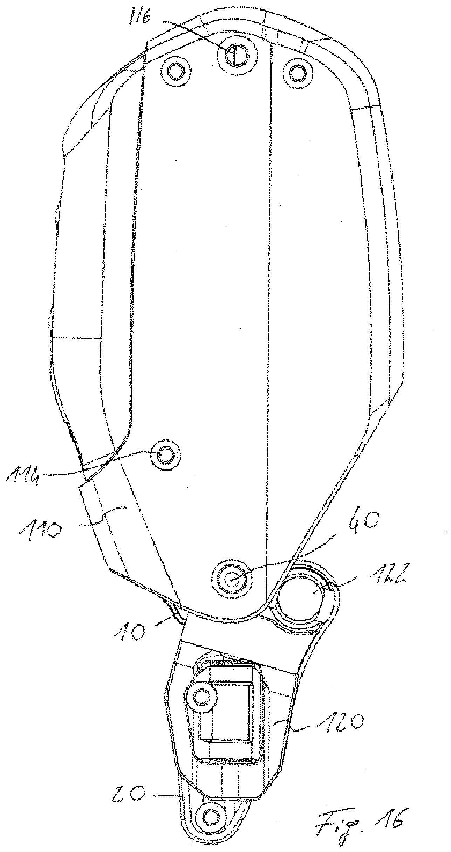

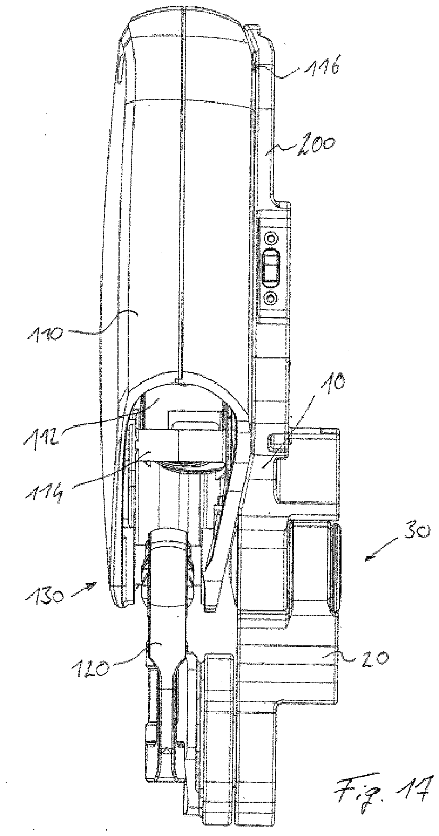

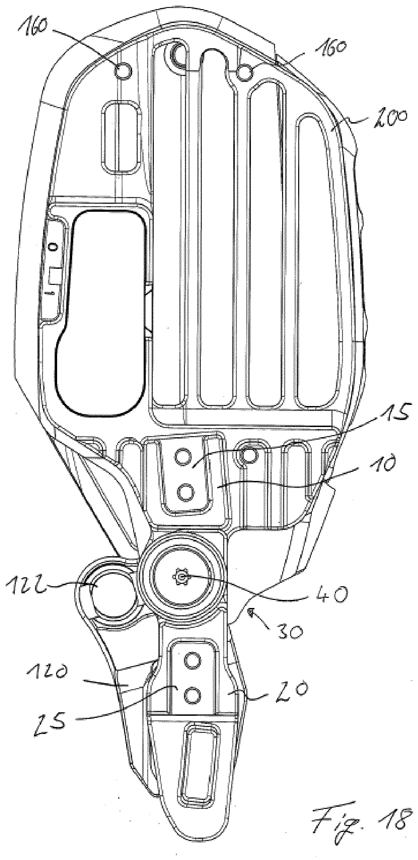

In der Frontalansicht gemäß Figur 15 sind der kompakte Aufbau des Orthesengelenkes sowie die schalenartige Ausgestaltung des oberen Stützelementes 110 zu erkennen. Die Lateralansicht gemäß Figur 16 zeigt die oberen und unteren Befestigungsstellen 116, 114 sowie die Aufnahmeeinrichtung 122 in Gestalt eines Lagerauges an dem unteren Stützelement 120. Die Rückansicht gemäß Figur 17 verdeutlicht die hohle, gehäuseartige Ausgestaltung des oberen Stützelementes 110, wobei das obere Stützelement 110 so formstabil ausgebildet ist, dass eine ausreichende Kraftübertragung und Kraftaufnahme stattfinden kann. Die Innenansicht gemäß Figur 18 zeigt das Oberteil 10 mit einer sich daran proximal anschließenden, plattenförmigen Vergrößerung, um eine ausreichende Auflagefläche für die Befestigung des oberen Stützelementes 110 zu ermöglichen. Das Unterteil 20 mit dem Schienenkasten 25 ist mit dem unteren Stützelement 120 und der Aufnahme 122 für einen Lagerbolzen formschlüssig und reversibel über Schrauben verbunden.

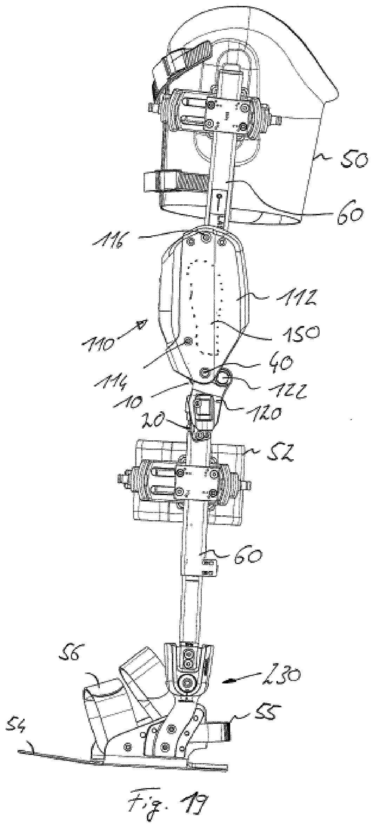

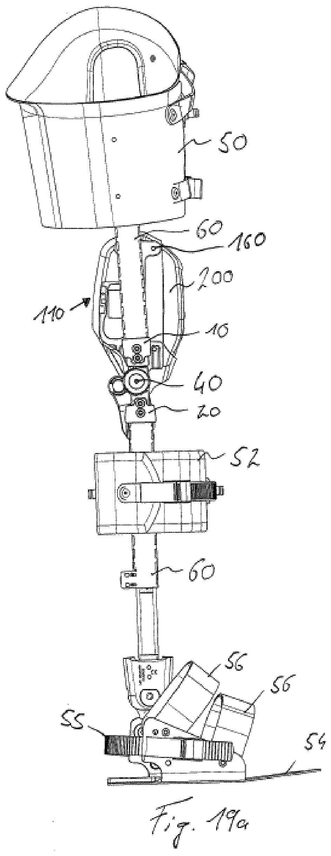

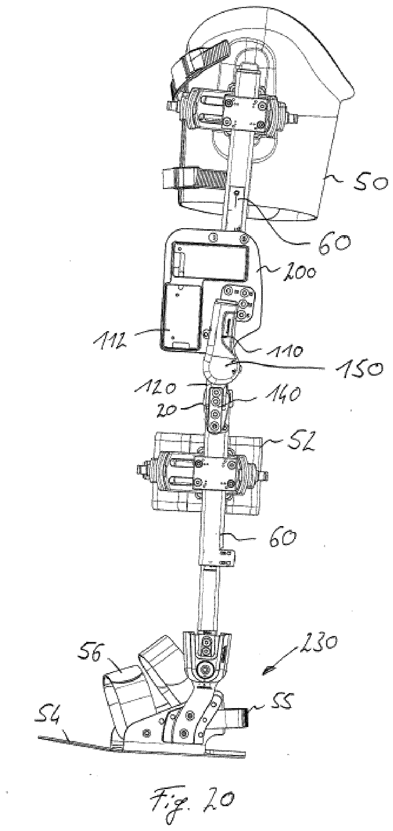

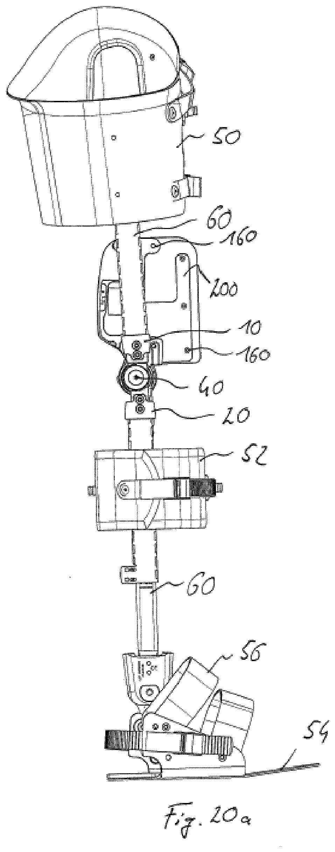

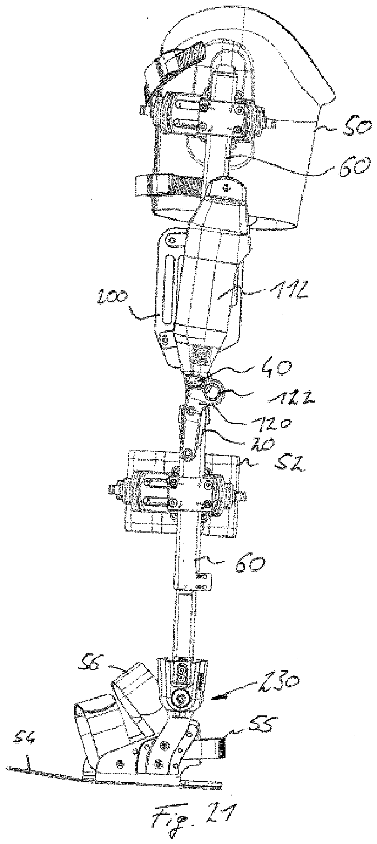

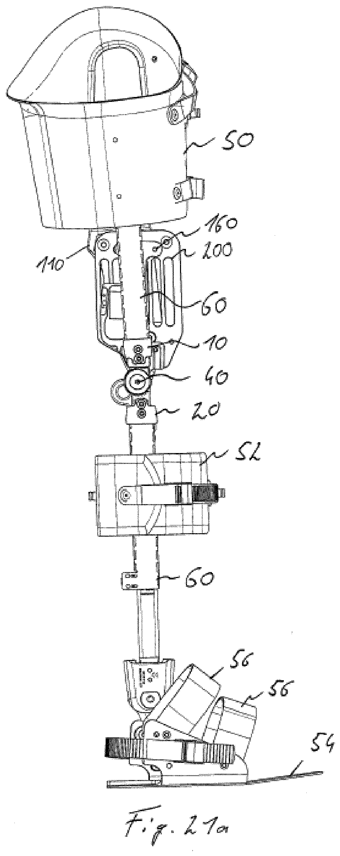

Die Figuren 19 bis 21 zeigen jeweils Seitenansichten eines Orthesensystems mit einem gleichen Grundaufbau, der im Wesentlichen dem Aufbau einer Orthese gemäß der Figuren 1, 2 und 3 entspricht. In der Figur 19 ist eine Variante der Ausführungsform gemäß Figur 3 gezeigt, in der Figur 20 ist eine Variante der Ausführungsform gemäß Figur 6 gezeigt und Figur 21 zeigt eine Variante der Ausführungsform gemäß Figur 11. Allen Orthesen gemeinsam ist der Grundaufbau mit Aufnahmeeinrichtungen 50, 52 und 54, wie er anhand der Figur 3 beschrieben wurde, wobei jeweils noch Gurte 56 zur Befestigung und Festlegung des Fußes auf der Fußplatte 54 hinzugekommen sind. Darüber hinaus ist ein Fersenbügel 55 an dem Fußteil angeordnet, sodass ein auf der Fußplatte 54 aufgesetzter Fuß über die beiden Befestigungsgurte 56 sowohl auf der Fußplatte 54 als auch gegen den Fersenbügel 55 gedrückt oder daran gehalten ist. An dem Knöchelgelenk 230, an dem auch Funktionselemente wie einstellbare Anschläge oder Federn angeordnet sein können, schließt sich in proximaler Richtung die Unterschenkelschiene 60 an, an der eine Unterschenkelschale 52 einstellbar sowohl in der Höhe als auch in anterior-posterior-Richtung sowie in einer Winkelstellung relativ zu der Längserstreckung der Unterschenkelschiene 60 befestigt ist. Um die Kniegelenksachse 40 herum ist das Oberteil 10 relativ zu dem Unterteil 20 schwenkbar ausgebildet. Proximal zu dem Oberteil 10 erstreckt sich die Oberschenkelschiene 60, an der die Oberschenkelschale 50 befestigt ist. Die Befestigung der Oberschenkelschale 50 an der Oberschenkelschiene 60 erfolgt korrespondierend zu der Befestigung der Unterschenkelschale 52 an der Unterschenkelschiene 60 in einem Adapter, der entlang der Längserstreckung der Oberschenkelschiene 60 verlagerbar ist und über den eine Einstellung sowohl der Winkelstellung als auch in anterior-posterior-Richtung und in medial-lateral-Richtung, also von der Oberschenkelschiene weg oder dazu hin, ermöglicht wird.

Dieser Grundaufbau ist bei dem System, das in den Figuren 19 bis 21 gezeigt ist, gleich. Die Orthesen unterscheiden sich durch unterschiedliche Funktionselemente 150, von denen nur eines sichtbar ist. Bei der Ausführungsform gemäß Figur 19 ist in der Aufnahme 112 eine hydraulische Dämpfereinrichtung als Funktionselement 150 angeordnet, die über die Aufnahme 112 mit dem Oberteil 10 gekoppelt und beispielsweise die Aufnahme 122 mit dem Unterteil 20 über das untere Stützelement 120 verbunden ist.

Bei der Ausführungsform gemäß Figur 20 ist an dem Aufbau gemäß der Figur 19 statt eines Hydraulikdämpfers eine angetriebene Verriegelungs- und Freigabeeinrichtung als ein Funktionselement 150 angeordnet, der an dem oberen Stützelement 110 befestigt ist. Alternativ dazu kann ein Antrieb als Funktionselement in der Aufnahme 112 gemäß Figur 19 angeordnet sein. Der Antrieb ermöglicht die Einleitung oder zumindest Unterstützung einer Verschwenkbewegung um die Schwenkachse 40, die sich in der Darstellung der Figur 20 hinter dem Antrieb befindet.

In der Figur 21 ist die Anordnung eines computergesteuerten Hydraulikdämpfers, wie er in der Figur 11 bereits gezeigt ist, an der Orthese dargestellt. Der Hydraulikdämpfer als Funktionselement ist in der Aufnahme 112 angeordnet und über ein Kopplungselement mit der Aufnahme 122 an dem unteren Stützelement 120 koppelbar.

An dem Grundaufbau mit den Aufnahmeeinrichtungen 50, 52, 54 und 56, den Schienen 60 und der ersten Gelenkeinrichtung 10, 20 ändert sich in den Ausführungsformen gemäß der Figuren 19 bis 21 nichts, die zweite Gelenkeinrichtung 130, die über das jeweilige obere und untere Stützelement 110, 120 mit den daran angeordneten Funktionselementen 150 wie Hydraulikdämpfer, Verriegelungs- und Freigabeeinrichtung, motorischer Antrieb oder computergesteuerten Hydraulikdämpfungssystem sind so ausgebildet, dass sie Befestigungseinrichtungen aufweisen, die es ermöglichen, die unterschiedlichen, als Module ausgebildeten zweiten Gelenkeinrichtungen 130 an dem Grundaufbau und der ersten Gelenkeinrichtung 30 mit dem Oberteil 10 und Unterteil 20 festzulegen. Dadurch kann beispielsweise bei einem sich verschlechternden Gesundheitszustand von einem einfachen Hydraulikdämpfersystem, wie es in der Figur 19 gezeigt ist, über ein computergesteuertes Hydraulikdämpfersystem gemäß Figur 21 auf ein motorisch unterstütztes, aktives Orthesensystem oder eine Orthese mit eine Freigabe- und Verriegelungseinrichtung gemäß Figur 20 angepasst werden, oder aber, bei einer Verbesserung des Gesundheitszustandes, von einem angetriebenen System über ein adaptivgesteuertes Orthesensystem gemäß Figur 21 zu einem passiven Orthesensystem gemäß Figur 19 und dann zu einem Verriegelungs- und Freigabesystem gemäß Figur 20 übergegangen werden.

Die Figuren 19a bis 21a zeigen jeweils mediale Ansichten der Ausführungsformen gemäß der Figuren 19 bis 21. In der medialen Ansicht ist zu erkennen, dass an der Oberschenkelschiene 60 eine Befestigungseinrichtung 160 angeordnet oder ausgebildet ist, an der das obere Stützelement 110 über Schrauben, Bolzen oder ähnliches festlegbar ist. Eine korrespondierende Befestigung wird über das untere Stützelement 120 entweder unmittelbar an dem Unterteil 20 oder an der Unterschenkelschiene 60 erfolgen. Alle Funktionselemente 150 oder alle Stützelemente 110, 120 weisen korrespondierende Befestigungsstellen 114, 116 auf, die beispielsweise als Bohrungen oder Stifte ausgebildet sind und mit den Befestigungseinrichtungen 160 fluchten oder mit ihnen in Eingriff bringbar sind, um die zweite Gelenkeinrichtung 130 an der ersten Gelenkeinrichtung 30 durch eine Kopplung zwischen den Stützelementen und dem Oberteil bzw. Unterteil zu erreichen. Die Befestigungseinrichtung 160 kann auch unmittelbar an dem Oberteil 10 oder dem Unterteil 20 angeordnet sein.

## Patentansprüche

1. Orthese mit einem Oberteil (10) und einem Unterteil (20), die über eine erste Gelenkeinrichtung (30) verschwenkbar um eine Schwenkachse (40) miteinander gekoppelt sind, wobei an dem Oberteil (10) und/oder Unterteil (20) Einrichtungen (50, 52, 54) zur Aufnahme eines Körperteils oder einer Gliedmaße oder zur Festlegung der Orthese (1) an einem Körperteil oder einer Gliedmaße angeordnet sind, **dadurch gekennzeichnet, dass** das Oberteil (10) und das Unterteil (20), im angelegten Zustand, jeweils nur einseitig medial oder lateral neben der zu versorgenden Gliedmaße angeordnet ist und an dem Oberteil (10) einseitig ein oberes Stützelement (110) und an dem Unterteil (20) einseitig ein unteres Stützelement (120) festgelegt sind, die über eine zweite Gelenkeinrichtung (130) schwenkbar um die Schwenkachse (40) miteinander verbunden sind und dass an dem Oberteil (10) und/oder dem Unterteil (20) eine Lagerplatte (200) befestigt oder ausgebildet ist, die zumindest eine Befestigungseinrichtung (160) zur Festlegung der zweiten Gelenkeinrichtung (130) aufweist.

2. Orthese nach Anspruch 1, **dadurch gekennzeichnet, dass** die erste Gelenkeinrichtung (30) und die zweite Gelenkeinrichtung (130) lateral zueinander beabstandet angeordnet sind.

3. Orthese nach Anspruch 1 oder 2, **dadurch gekennzeichnet, dass** die Stützelemente (110, 120) lösbar über die zweite Gelenkeinrichtung (130) miteinander verbunden sind.

4. Orthese nach einem der voranstehenden Ansprüche, **dadurch gekennzeichnet, dass** an dem Oberteil (10) und dem Unterteil (20) Befestigungseinrichtungen (15, 25) zur lösbaren Befestigung von Schienen (60) oder Einrichtungen (50, 52, 54, 56) zur Aufnahme eines Körperteils oder einer Gliedmaße ausgebildet oder angeordnet sind.

5. Orthese nach einem der voranstehenden Ansprüche, **dadurch gekennzeichnet, dass** an dem oberen Stützelement (110) und dem unteren Stützelement (120) Aufnahmen (112, 122) für einen Dämpfer, eine Freigabe- und Verriegelungseinrichtung, einen Antrieb, eine Steuerungseinrichtung, einen Federspeicher, einen Vorbringer und/oder einen Sensor angeordnet oder ausgebildet sind.

6. Orthese nach einem der voranstehenden Ansprüche, **dadurch gekennzeichnet, dass** die Stützelemente (110, 120) über die Lagerplatte (200) lösbar an dem Oberteil (10) und dem Unterteil (20) befestigt sind.

7. Orthese nach einem der voranstehenden Ansprüche, **dadurch gekennzeichnet, dass** das obere und/oder untere Stützelement (110, 120) zu der Schwenkachse (40) winkeleinstellbar ausgebildet ist.

8. Orthese nach einem der voranstehenden Ansprüche, **dadurch gekennzeichnet, dass** die Orthese als knieübergreifende Orthese, KAFO oder Knöchel-Fuß-Orthese ausgebildet ist.

9. Orthese nach einem der voranstehenden Ansprüche, **dadurch gekennzeichnet, dass** die Stützelemente (110, 120) kraftübertragend und momentenübertragend an dem Oberteil (10) und dem Unterteil (20) festgelegt sind.

10. Orthese nach einem der voranstehenden Ansprüche, **dadurch gekennzeichnet, dass** die Stützelemente (110, 120) aus Metall oder einem faserverstärkten Kunststoff ausgebildet sind.

11. Orthese nach einem der Ansprüche 1 bis 10, **dadurch gekennzeichnet, dass** zumindest eines der Stützelemente (110; 120) als ein Funktionselement (150) ausgebildet ist und/oder an zumindest einem Stützelement (110, 120) ein solches Funktionselement (150) befestigt ist.

12. System aus einer Orthese nach einem der voranstehenden Ansprüche und einer Vielzahl unterschiedlicher Funktionselemente (150), die an denselben Befestigungseinrichtungen (160) an der Lagerplatte (200) festlegbar sind.

13. System nach Anspruch 12, **dadurch gekennzeichnet, dass** alle Funktionselemente (150) oder Stützelemente (110, 120) Befestigungsstellen (114, 116) aufweisen, die korrespondierend zu einer Befestigungseinrichtung (160) für die Befestigungsstellen (114, 116) zur Befestigung an dem Oberteil (10) und dem Unterteil (20) angeordnet sind.

14. System nach Anspruch 12 oder 13, **dadurch gekennzeichnet, dass** zwischen dem jeweiligen Funktionselement (150) und dem Oberteil (10) und/oder Unterteil (20) oder der Lagerplatte (200) zumindest ein Distanzelement (90, 95) angeordnet ist.

15. System nach einem der Ansprüche 12 bis 14, **dadurch gekennzeichnet, dass** das Funktionselement (150) auswechselbar an der Lagerplatte (200) festgelegt ist.

16. System nach einem der Ansprüche 12 bis 15, **dadurch gekennzeichnet, dass** das obere Stützelement (110) und das untere Stützelement (120) zu einem Modul montiert sind.

17. Verfahren zum Anpassen einer Orthese nach einem der Ansprüche 1 bis 11 an einen Patienten, an dem die Orthese über die Einrichtungen (50, 52, 54) zur Aufnahme oder zur Festlegung eines Körperteils oder einer Gliedmaße an dem Körperteil oder der Gliedmaße befestigt wird, mit den Schritten:
- Auswählen einer zweiten Gelenkeinrichtung (130) aus einer Vielzahl an verschiedenen zweiten Gelenkeinrichtungen (130), und
- Verbinden eines oberen und unteren Stützelementes (110, 120), die über die ausgewählte zweite Gelenkeinrichtung (130) miteinander verbunden sind, mit dem Oberteil (10) und dem Unterteil (20) der ersten Gelenkeinrichtung (30).

18. Verfahren nach Anspruch 17, **dadurch gekennzeichnet, dass** eine erste Gelenkeinrichtung (30) aus einer Vielzahl an verschiedenen ersten Gelenkeinrichtungen (30) ausgewählt und mit den Einrichtungen (50, 52, 54) zur Aufnahme oder Festlegung eines Körperteiles oder Gliedmaße verbunden wird.

## Claims

1. An orthosis with an upper part (10) and a lower part (20), which are coupled to each other via a first joint device (30) in such a way as to be pivotable about a pivot axis (40), wherein devices (50, 52, 54) for receiving a body part or a limb or for securing the orthosis (1) to a body part or a limb are arranged on the upper part (10) and/or lower part (20), **characterized in that** the upper part (10) and the lower part (20) are, in the applied state, in each case arranged on only one side medially or laterally next to the limb to be fitted and an upper support element (110) is secured to the upper part (10) on one side and a lower support element (120) is secured to the lower part (20) on one side, which support elements are connected to each other via a second joint device (130) in such a way as to be pivotable about the pivot axis (40), and that a bearing plate (200) having at least one fastening device (160) for securing the second joint device (130) is fastened to or formed on the upper part (10) and/or the lower part (20).

2. The orthosis as claimed in claim 1, **characterized in that** the first joint device (30) and the second joint device (130) are arranged laterally and spaced apart from each other.

3. The orthosis as claimed in claim 1 or 2, **characterized in that** the support elements (110, 120) are connected to each other releasably via the second joint device (130).

4. The orthosis as claimed in one of the preceding claims, **characterized in that** fastening devices (15, 25) for the releasable fastening of rails (60) or devices (50, 52, 54, 56) for receiving a body part or a limb are formed or arranged on the upper part (10) and the lower part (20).

5. The orthosis as claimed in one of the preceding claims, **characterized in that** receptacles (112, 122) for a damper, an enabling and locking device, a drive, a control device, a spring store, an extension assist and/or a sensor are arranged or formed on the upper support element (110) and the lower support element (120).

6. The orthosis as claimed in one of the preceding claims, **characterized in that** the support elements (110, 120) are fastened releasably to the upper part (10) and the lower part (20) via the bearing plate (200).

7. The orthosis as claimed in one of the preceding claims, **characterized in that** the upper and/or lower support element (110, 120) is designed to be adjustable in terms of angle to the pivot axis (40).

8. The orthosis as claimed in one of the preceding claims, **characterized in that** the orthosis is designed as a cross-knee orthosis, KAFO or ankle-foot orthosis.

9. The orthosis as claimed in one of the preceding claims, **characterized in that** the support elements (110, 120) are secured to the upper part (10) and the lower part (20) to transmit force and moment.

10. The orthosis as claimed in one of the preceding claims, **characterized in that** the support elements (110, 120) are made from metal or a fiberreinforced plastic.

11. The orthosis as claimed in one of claims 1 through 10, **characterized in that** at least one of the support elements (110; 120) is designed as a functional element (150), and/or such a functional element (150) is fastened to at least one support element (110, 120).

12. A system composed of an orthosis as claimed in one of the preceding claims and of a multiplicity of different functional elements (150), which can be secured to the bearing plate (200) at the same fastening devices (160).

13. The system as claimed in claim 12, **characterized in that** all of the functional elements (150) or support elements (110, 120) have fastening locations (114, 116) which are arranged corresponding to a fastening device (160) for the fastening locations (114, 116) for fastening to the upper part (10) and the lower part (20).

14. The system as claimed in claim 12 or 13, **characterized in that** at least one spacer element (90, 95) is arranged between the respective functional element (150) and the upper part (10) and/or lower part (20) or the bearing plate (200).

15. The system as claimed in one of claims 12 through 14, **characterized in that** the functional element (150) is secured exchangeably to the bearing plate (200).

16. The system as claimed in one of claims 12 through 15, **characterized in that** the upper support element (110) and the lower support element (120) are assembled to form a module.

17. A method for adapting an orthosis as claimed in one of claims 1 through 11 to a patient, on whom the orthosis is fastened to a body part or limb via the devices (50, 52, 54) for receiving or for securing a body part or a limb, said method having the steps of:
- selecting a second joint device (130) from a multiplicity of different second joint devices (130), and
- connecting an upper and lower support element (110, 120), which are connected to each other via the selected second joint device (130), to the upper part (10) and the lower part (20) of the first joint device (30).

18. The method as claimed in claim 17, **characterized in that** a first joint device (30) is selected from a multiplicity of different first joint devices (30) and is connected to the devices (50, 52, 54) for receiving or securing a body part or limb.

## Revendications

1. Orthèse comprenant une partie supérieure (10) et une partie inférieure (20), qui sont couplées l'une à l'autre par l'intermédiaire d'un premier dispositif d'articulation (30) de manière à pouvoir pivoter autour d'un axe de pivotement (40), des dispositifs (50, 52, 54) destinés à recevoir une partie du corps ou un membre ou à immobiliser l'orthèse (1) sur une partie du corps ou un membre étant disposés sur la partie supérieure (10) et/ou sur la partie inférieure (20),
**caractérisée en ce que**
la partie supérieure (10) et la partie inférieure (20), à l'état appliqué, ne sont disposées chacune que sur un côté, médial ou latéral, à côté du membre à soigner, et un élément de soutien supérieur (110) est immobilisé sur un côté sur la partie supérieure (10), et un élément de soutien inférieur (120) est immobilisé sur un côté sur la partie inférieure (20), lesquels sont reliés l'un à l'autre par un deuxième dispositif d'articulation (130) de manière à pouvoir pivoter autour de l'axe de pivotement (40), et
**en ce qu'**une plaque de support (200) est fixée ou réalisée sur la partie supérieure (10) et/ou sur la partie inférieure (20), qui comprend au moins un dispositif de fixation (160) pour immobiliser le deuxième dispositif d'articulation (130).

2. Orthèse selon la revendication 1,
**caractérisée en ce que** le premier dispositif d'articulation (30) et le deuxième dispositif d'articulation (130) sont disposés en étant espacés latéralement l'un de l'autre.

3. Orthèse selon la revendication 1 ou 2,
**caractérisée en ce que** les éléments de soutien (110, 120) sont reliés entre eux de manière amovible par l'intermédiaire du deuxième dispositif d'articulation (130).

4. Orthèse selon l'une des revendications précédentes,
**caractérisée en ce que** des dispositifs de fixation (15, 25) sont réalisés ou disposés sur la partie supérieure (10) et sur la partie inférieure (20) pour la fixation amovible de rails (60) ou de dispositifs (50, 52, 54, 56) destinés à recevoir une partie du corps ou un membre.

5. Orthèse selon l'une des revendications précédentes,
**caractérisée en ce que** des logements (112, 122) pour un amortisseur, un dispositif de libération et de verrouillage, un entraînement, un dispositif de commande, un accumulateur à ressort, un dispositif d'avancement et/ou un capteur sont disposés ou réalisés sur l'élément de soutien supérieur (110) et sur l'élément de soutien inférieur (120).

6. Orthèse selon l'une des revendications précédentes,
**caractérisée en ce que** les éléments de soutien (110, 120) sont fixés de manière amovible à la partie supérieure (10) et à la partie inférieure (20) par l'intermédiaire de la plaque de support (200).

7. Orthèse selon l'une des revendications précédentes,
**caractérisée en ce que** l'élément de soutien supérieur et/ou inférieur (110, 120) est réalisé de manière à présenter un angle réglable par rapport à l'axe de pivotement (40).

8. Orthèse selon l'une des revendications précédentes,
**caractérisée en ce que** l'orthèse est conçue comme une orthèse coiffant le genou, une orthèse de genou-cheville-pied (KAFO) ou une orthèse de cheville-pied.

9. Orthèse selon l'une des revendications précédentes,
**caractérisée en ce que** les éléments de soutien (110, 120) sont immobilisés sur la partie supérieure (10) et sur la partie inférieure (20) de manière à transmettre les forces et les couples.

10. Orthèse selon l'une des revendications précédentes,
**caractérisée en ce que** les éléments de soutien (110, 120) sont réalisés en métal ou en matière plastique renforcée par des fibres.

11. Orthèse selon l'une des revendications 1 à 10,
**caractérisée en ce que** l'un au moins des éléments de soutien (110 ; 120) est réalisé sous la forme d'un élément fonctionnel (150), et/ou un tel élément fonctionnel (150) est fixé à au moins un élément de soutien (110, 120).

12. Système constitué d'une orthèse selon l'une des revendications précédentes et d'une pluralité d'éléments fonctionnels (150) différents qui peuvent être immobilisées sur la plaque de support (200) au niveau des mêmes dispositifs de fixation (160).

13. Système selon la revendication 12,
**caractérisé en ce que** tous les éléments fonctionnels (150) ou éléments de soutien (110, 120) présentent des emplacements de fixation (114, 116) qui sont disposés en correspondance avec un dispositif de fixation (160) pour les emplacements de fixation (114, 116) destinés à la fixation à la partie supérieure (10) et à la partie inférieure (20).

14. Système selon la revendication 12 ou 13,
**caractérisé en ce qu'**au moins un élément d'écartement (90, 95) est disposé entre l'élément fonctionnel respectif (150) et la partie supérieure (10) et/ou la partie inférieure (20) ou la plaque de support (200).

15. Système selon l'une des revendications 12 à 14,
**caractérisé en ce que** l'élément fonctionnel (150) est immobilisé de manière interchangeable sur la plaque de support (200).

16. Système selon l'une des revendications 12 à 15,
**caractérisé en ce que** l'élément de soutien supérieur (110) et l'élément de soutien inférieur (120) sont assemblés en un module.

17. Procédé d'adaptation d'une orthèse selon l'une des revendications 1 à 11 à un patient auquel l'orthèse est fixée au niveau d'une partie du corps ou d'un membre par l'intermédiaire des dispositifs (50, 52, 54) de réception ou d'immobilisation de ladite partie du corps ou dudit membre, comprenant les étapes consistant à :
- sélectionner un deuxième dispositif d'articulation (130) parmi une pluralité de deuxièmes dispositifs d'articulation différents (130), et
- relier un élément de soutien supérieur et un élément de soutien inférieur (110, 120), reliés l'un à l'autre par l'intermédiaire du deuxième dispositif d'articulation sélectionné (130), à la partie supérieure (10) et à la partie inférieure (20) du premier dispositif d'articulation (30).

18. Procédé selon la revendication 17,
**caractérisé en ce qu'**un premier dispositif d'articulation (30) est sélectionné parmi une pluralité de premiers dispositifs d'articulation (30) différents et est relié aux dispositifs (50, 52, 54) de réception ou d'immobilisation d'une partie du corps ou d'un membre.
